# DEMANDE DE BREVET EUROPEEN

(11) **EP 1 671 613 A1**
(43) Date de publication de la demande: **21.06.2006**
(21) Numéro de dépôt: 05292541.9
(22) Date de dépôt: 01.12.2005
(51) Int. Cl.: A61K 8/02, A61K 8/46, A61K 8/99, A61Q 3/00

(54) **Dispositif transunguéal**

(30) Priorité: 07.12.2004 FR 0452892
(71) Demandeur: L'ORÉAL, 75008 Paris (FR)
(72) Inventeur: Legendre, Jean-Yves, 75015 Paris (FR); Cavazzuti, Roberto, 75015 Paris (FR)
(74) Mandataire: Duvert, Sandra

(57) **Abrégé**

La présente invention a pour objet un dispositif transunguéal comprenant au moins un actif choisi parmi un extrait de bactérie filamenteuse non photosynthétique non fructifiante, une dialkylsulfone et leur mélange.

## Description

L'invention concerne un dispositif transungéal comprenant au moins un actif choisi parmi un extrait de bactérie filamenteuse non photosynthétique non fructifiante et une dialkylsulfone.

Il est bien connu que les ongles présentent, de façon fréquente, des défauts de structure et de consistance, ceux-ci pouvant être d'origine diverses et notamment liés au métabolisme propre de l'individu, à ses conditions de vie, à son mode d'alimentation, à son âge, à ses états de fatigue ou de surmenage.
Ces défauts peuvent également apparaître sous l'effet d'actions qui érodent, à la suite par exemple d'expositions prolongées ou répétées à des agents détergents, à des solvants, à des produits chimiques en particulier ménager, à des atmosphères chaudes ou froides, humides ou sèches, ou à des expositions aux rayonnements UV.
Ces défauts de structure et de consistance ont pour effet de rendre la surface des ongles inesthétique, ce qui peut être source de gêne et de désagréments multiples.

On connaît des produits comprenant des actifs susceptibles de renforcer les ongles qui sont généralement appliqués par voie topique sous forme de gels ou lotion ; toutefois, la diffusion du principe actif à travers la surface de l'ongle est très faible et la durée du traitement est particulièrement longue. De plus, ces formes de dosage topique ne permettent pas de maintenir le principe actif en contact avec l'ongle pendant une durée prolongée.
Une autre forme topique connue est le vernis à ongle mais le traitement des ongles par un vernis est assez contraignant car il est répétitif et demande un entretien de l'ongle avant chaque application.

Or le demandeur vient de montrer qu'un actif particulier choisi parmi un extrait de bactérie filamenteuse non photosynthétique non fructifiante et/ou une dialkylsulfone est capable de diffuser efficacement à travers l'ongle à l'aide d'un dispositif transunguéal.

L'invention a ainsi pour objet un dispositif transungéal comprenant au moins un actif choisi parmi un extrait de bactérie filamenteuse non photosynthétique non fructifiante, une dialkylsulfone et leur mélange.

Un tel dispositif permet, après application sur les ongles, de renforcer les ongles et en particulier de durcir les ongles, d'augmenter leur résistance à la cassure et/ou au dédoublement, d'augmenter l'épaisseur des ongles, et stimuler la pousse et/ou la repousse des ongles, en particulier des ongles mous, et améliorer ainsi leur aspect global. Il permet également de limiter la perte insensible en eau des ongles et de favoriser leur hydratation, permettant ainsi une meilleure pénétration de l'actif dans l'ongle. Par perte insensible en eau, on entend le pourcentage d'eau qui traverse les matières kératiniques (plus précisément le stratum cornéum) et s'évapore à leur surface.

Outre la biodisponibilité et le contact de l'actif avec l'ongle qui sont améliorés, le dispositif permet l'incorporation d'une quantité importante d'actif, supérieure à celle d'un vernis à ongles dans lequel la teneur en actif peut être limitée par les problèmes de solubilité de l'actif dans le milieu solvant.

De tels actifs sont connus notamment par les documents WO02/056858 ou EP0761204 pour l'extrait de bactérie et FR0216651 pour la dialkylsulfone.
Toutefois, leur mise en oeuvre sous forme de dispositif transunguéal pour renforcer les ongles et améliorer l'état général de l'ongle n'est nullement décrit dans ces documents.

L'invention concerne également l'utilisation d'un dispositif transungéal comprenant au moins un actif choisi parmi un extrait de bactérie filamenteuse non photosynthétique non fructifiante, une dialkylsulfone et leur mélange, pour renforcer les ongles et en particulier durcir les ongles et/ou augmenter leur résistance à la cassure et/ou au dédoublement et/ou augmenter l'épaisseur des ongles, et/ou stimuler la pousse et/ou la repousse des ongles.
L'invention a égalment pour objet l'utilisation d'un dispositif transungéal comprenant au moins un actif choisi parmi un extrait de bactérie filamenteuse non photosynthétique non fructifiante, une dialkylsulfone et leur mélange, pour limiter la perte insensible en eau des ongles, favoriser l'hydratation des ongles et/ou favoriser la pénétration de l'actif dans l'ongle.

L'invention concerne également un procédé de traitement cosmétique consistant à appliquer sur l'ongle un dispositif transunguéal tel que décrit ci-dessus.

### Actif

L'actif peut être choisi parmi un extrait de bactérie filamenteuse non photosynthétique non fructifiante, une dialkylsulfone et leur mélange.

### Extrait de bactérie

L'extrait de bactérie utilisable dans le cadre de l'invention est préparé de préférence à partir de bactéries filamenteuses non photosynthétiques telles que définies selon la classification du Bergey's Manual of Systematic Bacteriology (vol. 3, sections 22 et 23, 9°édition, 1989), parmi lesquelles on peut citer les bactéries appartenant à l'ordre des Beggiatoales, et plus particulièrement les bactéries appartenant aux genres *Beggiatoa, Vitreoscilla, Flexithrix ou Leucothrix.*
Parmi les bactéries utilisables, on peut citer par exemple :
*Vitreoscilla filiformis* (ATCC 15551)
*Vitreoscilla beggiatoïdes* (ATCC 43181)
*Beggiatoa alba* (ATCC 33555)
*Flexithrix dorotheae* (ATCC 23163)
*Leucothrix mucor* (ATCC 25107)
*Sphaerotilus natans* (ATCC 13338)
De préférence, on utilisera un extrait de *Vitreoscilla filiformis* (ATCC 15551).

Par 'extrait de bactérie' selon l'invention, on entend un extrait de la biomasse bactérienne ou toute fraction active dudit extrait, en particulier :
(i) des cellules de bactérie isolées du milieu de culture, qui ont été concentrées, par exemple par centrifugation (extrait cellulaire non stabilisé) ; ou
(ii) des cellules de bactérie concentrées (i), puis soumises à une opération de rupture des enveloppes des cellules de bactérie, par tout moyen connu de l'homme du métier, comme l'action d'ultrasons ou préférentiellement l'autoclavage (extrait cellulaire stabilisé). Par 'enveloppes', on entend paroi bactérienne et éventuellement les membranes sous-jacentes ;
(iii) le surnageant obtenu par filtration de l'extrait cellulaire stabilisé (ii),
ou toute fraction active dudit extrait. Par fraction 'active' dudit extrait, on entend toute fraction capable de renforcer et/ou améliorer les matières kératiniques selon l'invention.

Cette fraction active peut être obtenue par les méthodes classiques de fractionnement, telles que l'extraction en présence d'un solvant, la précipitation sélective ou l'ultrafiltration tangentielle (UFT) par exemple.

Ces extraits ou fractions peuvent être conservés par exemple par congélation desdits extraits ou desdites fractions et utilisés après décongélation.
On parlera plus simplement dans le reste de la description 'd'extrait cellulaire' de bactéries ((i) et (ii)) ou de 'surnageant' dudit extrait (iii).

Et de préférence, on utilisera dans le cadre de l'invention un 'extrait cellulaire' de bactéries (sous la forme (i) ou (ii)) ou une fraction active dudit extrait.

Pour préparer l'extrait de bactérie selon l'invention, on peut cultiver lesdites bactéries selon les méthodes connues de l'homme du métier, ou se reporter en particulier à la description de la demande de brevet WO-A-93-00741. On obtient un extrait cellulaire dont on peut séparer le surnageant par exemple par filtration et centrifugation. L'extrait peut être utilisé sous forme aqueuse ou sous forme lyophilisée. Le protocole est décrit plus en détail à l'exemple 1 ci-après.
Cet extrait de bactérie peut être refractionné et utilisé pur ou dilué à différentes concentrations.

### Dialkylsulfone

La dialkylsulfone présente avantageusement la formule (I) suivante :

R₁-SO₂-R₂ (I)

dans laquelle R₁ et R₂ identiques ou différents représentent un groupe alkyle linéaire ou ramifié de 1 à 4 atomes de carbone, dans une composition cosmétique de soin des ongles, appliquée sur les ongles, pour favoriser la pousse des ongles.
Le groupe alkyle de R₁ et R₂ est choisi indépendamment parmi les groupes alkyle linéaire et ramifié de 1 à 4 atomes de carbone, tels que les groupes méthyle, éthyle, isopropyle, n-propyle, n-butyle, isobutyle et tertiobutyle.
De préférence, R₁ et R₂ sont identiques.
De préférence encore, R₁ et R₂ représentent tous deux un groupe méthyle et le composé de formule (I) est alors la diméthylsulfone ou méthylsulfonylméthane (MSM).
Les composés de formule (I) et, en particulier le MSM, sont des composés connus. Le MSM a l'avantage d'être un produit sans odeur, blanc, cristallin, qui a un point de fusion de 109°C et un point d'ébullition de 238°C.
Le MSM est présent dans la plupart des végétaux verts, du microplancton et des algues. C'est un composé naturel que l'on trouve dans les tissus nerveux, la peau, les cheveux et les articulations.

L'actif peut être présent en une quantité allant de 0,1 % à 90 % en poids par rapport au poids total de la matrice du dispositif, telle que définie plus loin, préférentiellement allant de 0,5 à 50% en poids et encore plus préférentiellement de 1 à 30 % en poids.

### Dispositif

Par 'dispositif transunguéal', ou système de délivrance transunguéal au sens de l'invention on entend tout système permettant une libération active ou passive de la substance active permettant son transfert dans la matrice de l'ongle.

Parmi les dispositifs transunguéals applicables à l'invention, on peut citer par exemple :
- des patchs, c'est-à-dire tout système de délivrance d'actif présentant une structure composite sous forme de couches qui, par application sur l'ongle, assure la libération du produit actif à travers l'ongle. On peut citer par exemple :
   - des patchs à libération contrôlée avec une matrice polymérique hydrophobe tels que décrits par exemple dans le document FR 2 738 744 ;
   - des patchs de type réservoir contenant un réservoir de substance active, une membrane de diffusion et une couche adhésive
   - des patchs à effet de champ magnétique favorisant la pénétration des actifs dans la peau tels que décrits par exemple dans FR 2 791 750 ;
   - des patchs à adhérence optimisée, comportant une couche de polymère hydrophobe fixée sur une couche support et contenant des particules de composé actif, des particules d'huile et des particules d'un agent hydro-absorbant tels que décrits par exemple dans FR2 761 889 ;
   - des gels patchs à forte teneur en eau, de type hydrogel, notamment à base d'au moins un hydrocolloïde et/ou de polysaccharides, de protéines, de polyacrylamide, d'acide polyacrylique, de copolymères d'acide acrylique et d'amidon, de polyvinylpyrrolidone ou de dérivés d'acide polyvinylique, et présentant une adhésion de contact, similaire à celle résultant d'un effet ventouse.
   - Des gels patchs hydrophobes, aussi appelés organogels ou oléogels à base de polyéthylène, de silicones ou de copolymère de styrène et d'isoprène ou de copolymères de styrène et butadiène. Ces systèmes ont l'avantage de pouvoir contenir de fortes proportions de composés hydrophobes
   - Des gels patchs à base de de polyuréthanes segmentés (SPUG) dont le caractère lipophile ou hydrophile peut être modifié par modification des chaînes polyoxyéthylénées (Y. Shikinami, Adhesive polymer gels for medical uses, Kagaku To Tokyo, 67 (4), 141-150, 1993)
- des feuilles ou pastilles décrites dans FR 2 512 651 ou FR 2 538 247 ;
- des films composites tels que décrits dans EP-A- 0 285 563 ou US 2002/0187181 ; avantageusement, on pourra utiliser un film composite comportant une couche réservoir constituée par une matrice formée d'un polymère de silicone à l'intérieur de laquelle sont disposées des inclusions constituées par la phase aqueuse active gélifiée grâce à au moins un agent gélifiant (FR 2 650 747 L'Oreal) ;
- des films fins solides à base de polymères naturels ou synthétiques qui appliqués sur l'ongle préalablement mouillé se dissolvent in situ; de tels films sont décrits dans les demande de brevet WO 02/053197, WO 02/05789, WO 2004/032859, WO 02/054997
- des films contenant un agent filmogène et un polymère adhésif hydrophile, aussi appelés, patch-non-patch, tels que décrits dans le brevet WO 02/30402
- des compositions à base de polymères ayant des propriétés adhésives capables de former un film que l'on retire après séchage (EP- 0 514 760, EP-0 826 364) ou une couche solide élastique et pliable retirée en appliquant un tissu ou un plastique adhésif (WO93/05893) ; ou des gels ou pâtes qui, après application sur l'ongle, sèchent pour donner un film que l'on retire par lavage, nettoyage ou arrachage ; on parlera généralement de 'vernis-patch' ou 'peel-patch'.

Mais on entend également tous les systèmes ou dispositifs augmentant l'absorption par un mécanisme actif (M.R. Prausnitz et al., Current status and future potential of transdermal drug delivery, Nature Rev., vol. 3, 115-124, 2004), comme par exemple :
- les systèmes d'injection sous pression ou sans-aiguille (Burkoth T.L. et al., *Transdermal and transmucosal powdered drug delivery*., Crit Rev Ther Drug Carrier Syst. 1999;16(4):331-84
- l'utilisation d'ultra-sons : Sonophorèse et phonophorèse (Joshi A. and Raje J., *Sonicated transdermal drug transport,* J. Control. Release, 83 (1), 13-22, 2002)
- l'utilisation de courants électriques : lontophorèse, electroporation, courant microfréquence (Riviere J.E. and Heit M.C., *Electrically-assisted transdermal drug delivery*, Pharm. Res. 14 (6), 687-697, 1997) (brevets US 6,148,232, WO 03/035167)
- l'utilisation d'ondes magnétiques : magnétophorèse (Murthy S.N., *Magnetophoresis: an approach to enhance transdermal drug diffusion.,* Pharmazie. 1999 May;54(5):377-9 )
- l'utilisation de micro-aiguilles ( Mc Allister D.V. et al., *Microfabricated microneedles for gene and drug delivery,* Annu. Rev. Biomed. Eng., 2, 289-313, 2000) (brevet US 6,451,240)

Ces systèmes de délivrance transunguéal seront formulés selon des techniques connues de l'homme du métier pour assurer le taux et la vitesse de diffusion adaptée aux actifs présents dans la composition.

Les systèmes de délivrance transunguéal contiendront les excipients adaptés à leur formulation connus de l'homme du métier et notamment au moins un composé choisi parmi les gélifiants, les polymères adhésifs, les stabilisants, les colorants, les pigments, les charges, les solvants, les ions et leur mélange.

Avantageusement, le dispositif selon l'invention se présente sous forme de patch comprenant un support souple et une matrice, de préférence adhésive, comprenant l'actif.
Par « matrice » ou « couche adhésive matricielle », on entend au sens de la présente invention un mélange d'au moins actif tel que défini précédemment , à l'état dispersé ou solubilisé, de façon homogène, dans un adhésif biocompatible et sensible à la pression, mélange qui peut, en outre, éventuellement contenir d'autres additifs.
Dans le cas d'un dispositif mono-couche, cette couche matricielle possède une surface directement en contact avec le support et une deuxième surface directement en contact avec le revêtement extérieur amovible à ôter lors de l'application du patch sur la zone à traiter.
Lors de l'utilisation, ce revêtement extérieur amovible est retiré de ladite surface de la couche matricielle qui est alors appliquée directement sur l'ongle.
Le principe actif diffuse à travers l'adhésif et ses mélanges le cas échéant, jusqu'à la surface de l'ongle.

L'adhésif au sens de la présente description, désigne un polymère ou mélange de polymères chimiquement inertes vis-à-vis des différents composants de la matrice dont notamment le ou les actifs.
La matrice comprend ainsi au moins un polymère. Le polymère peut être choisi parmi les polymères naturels ou synthétiques, de préférence hydrophiles, capables de gélifier en présence d'eau et de présenter un pouvoir adhésif, et de préférence adhésifs sensibles à la pression, c'est-à-dire des matériaux qui adhèrent instantanément à la peau sous l'application d'une faible pression.

Selon un mode de réalisation, le dispositif selon l'invention est un gel-patch hydrophile ou hydrogel dont la matrice comprend au moins un polymère hydrophile. De tels dispositifs favorisent l'hydratation de l'ongle et, de ce fait, la pénétration des actifs dans l'ongle.

Ces polymères hydrophiles seront préférentiellement choisis parmi les polymères et copolymères de vinylpyrrolidone, l'acide acrylique, les sels, dérivés et co-polymères d'acide acrylique et leurs mélanges. On peut en particulier citer les polymères acryliques vendus sous la dénomination commerciale « GELVA » par la société Monsanto ou sous la dénomination commerciale « DURO-TAK » par la société National Starch ou sous la dénomination « ACRONAL » par la société BASF, le polyacrylate de sodium tel que par exemple celui commercialisé sous la référence Aronvis S par la société NIHON JUNYAKU.

Le polymère peut être présent en une proportion allant de 5 à 60% en poids par rapport au poids total de la matrice, de préférence de 10 à 40% en poids.

La matrice comprend avantageusement une forte teneur en eau, pouvant par exemple aller de 30 à 90% en poids par rapport au poids total de la matrice.

Outre le ou les polymères et l'eau, la matrice hydrogel peut contenir aussi un solvant choisi parmi les alcools primaires tels que l'éthanol et l'isopropanol ou un ou plusieurs polyols qui sont des composés comportant au moins deux fonctions hydroxyle. Comme polyols, on peut citer par exemple la glycérine, le propylène glycol, le butylène glycol, l'hexylène glycol, les polyéthylène glycols, le dipropylène glycol, les alcools polyvinyliques et leurs mélanges. La quantité de solvant peut aller par exemple de 1 à 40 % en poids et de préférence de 5 à 30 % en poids par rapport au poids total de l'hydrogel.

La matrice peut également comprendre un ou plusieurs hydrocolloïdes ou gélifiants hydrophiles.
Ces composés peuvent être par exemple choisis dans le groupe formé par :
- la gomme de gellane ;
- la cellulose ou ses dérivés comme la carboxyméthylcellulose, l'hydroxypropyl-cellulose, la méthylcellulose, l'hydroxypropylméthylcellulose ou l'hydroxyéthyl-cellulose ainsi que les celluloses modifiées notamment par greffage de groupement alkyle ;
- les extraits d'algue tels que l'agar-agar, les carraghénanes, les alginates ;
- les extraits de graines tels que la gomme de caroube, la gomme de guar, les gommes de guar modifiées notamment par greffage de groupement alkyle ;
- les exsudats de plantes tels que la gomme arabique, la gomme karaya, la gomme adragante, la gomme de gatty ;
- les exsudats de micro-organismes tels que la gomme de xanthane ;
- les extraits de fruits tels que les pectines ;
- les dérivés du silicium tels que les hectorites synthétiques comme les produits "Laponite RD et RDS" vendus par la société WAVERLY, les silicates d'aluminium et de magnésium comme le produit "Veegum" vendu par la société VANDERBILT,
- les produits du type POLYCARE® , commercialisé par la société Rhone-Poulenc.
ou un mélange de ces composés.
L'hydrocolloïde peut être présent en une teneur allant de 5 à 40 % en poids et de préférence de 10 à 30 % en poids par rapport au poids total de la matrice.

La matrice peut en particulier comprendre au moins un accélérateur de pénétration, notamment choisi parmi les mono- et di-alcools non cycliques, l'acétate d'éthyle, l'acétate de butyle, le myristate d'isopropyle, les acides gras, les phospholipides, les terpènes, l'azone et dérivés, le propylène glycol et dérivés glycoliques, les cyclodextrines, l'octylsalicylate, le cyclopentadecanolide, les polysorbates, la polyvinylpyrrolidone et dérivés, cette liste n'étant pas limitative. L'accélérateur de pénétration peut être présent en une teneur allant de 0,01 % à 10 % en poids par rapport au poids de la matrice.

Ces agents seront choisis selon leurs propriétés propres afin de moduler l'adhésion et la viscosité de la matrice. Cette dernière pourra en outre renfermer des agents hydratants, tels que le glycerol, dans une proportion allant de 0,1 à 30%, préférentiellement de 1 à 20% en poids par rapport au poids de la matrice. En outre, la matrice adhésive pourra comprendre un ou des additifs choisis parmi les conservateurs, les colorants, les pigments, les charges, les parfums, les stabilisants, les initiateurs de polymérisation, les milieux solvant aqueux ou organique, les gélifiants, les ions et leurs mélanges

Le support du dispositif transunguéal est la partie du patch visible lorsqu'il est appliqué sur l'ongle
Il permet de maintenir le principe actif au contact de l'ongle pour accroître la diffusion du principe actif à l'intérieur de l'ongle.
Le support peut être plus ou moins souple (il présente de préférence une flexibilité suffisante pour s'adapter au mieux à la surface de l'ongle même dans le cas de surface irrégulière), non soluble et peut être tissé ou non-tissé ou encore être en matériau plastique choisi préférentiellement parmi le polyuréthane (commercialisé par exemple sous les marques BAYDUR®, DALTOFLEX®, UROFLEX®, HYPERLAST®, polyuréthane élastomère thermoplastique (DESMOPAN®, ESTANE®, LASTANE®, TEXIN®), élastomère thermoplastique SBS (styrène-butadiène-styrène) (CARIFLEX®, KRATON ®, SOLPRENE®), élastomère thermoplastique SEBS (Styrène-ethylène-butylène-styrène) (KRATON®), EVA (Ethylène-Vinyl acétate) (ELVAX®, ESCORENE®, OPTENE®), élastomère thermoplastique coether ester (CEE TPE) (ARNITEL®, HYTREL®, RITEFLEX®), polyéthylenes, polypropylènes, silicones.

La structure du support non soluble peut soit être respirante, soit être occlusive, c'est-à-dire constituée d'une matière imperméable à l'air et/ou la vapeur d'eau, de façon à faciliter la pénétration de l'actif dans l'ongle.

Le dispositif transunguéal comprend généralement un revêtement extérieur amovible.
Par revêtement extérieur amovible, on entend un support protecteur en contact direct avec la couche matricielle adhésive qui est enlevé juste avant l'application sur la peau. Il doit donc être inerte chimiquement et imperméable vis-à-vis de la formule du patch.
A titre d'exemple de matériau pouvant convenir à titre de revêtement extérieur amovible, on peut citer notamment une feuille d'aluminium, un papier contenant une feuille de polyéthylène ou un polyester siliconé.

Selon un mode de réalisation, le patch selon l'invention peuvent être préparé selon des techniques conventionnelles telles que l'enduction.
Selon un mode de réalisation particulier, un patch hydrogel est préparé comme suit : les ingrédients actifs et les adjuvants sont dissous dans l'eau. Le ou les polymères adhésifs, les hydrocolloïdes sont dissous séparément dans l'eau. Les deux solutions aqueuses sont ensuite mélangées, éventuellement en chauffant le mélange. Celui-ci est alors enduit sur son support sous une épaisseur contrôlée à l'aide d'une machine d'enduction. Les patchs sont ensuite découpés et éventuellement stockés à l'air libre pour permettre une bonne réticulation du gel.

Le dispositif selon l'invention peut se présenter sous forme prédécoupée à la taille de l'ongle ou à découper avant ou après son application, selon la taille et la forme désirée.

### EXEMPLES

### Exemple 1 : Préparation des extraits de Vitreoscilla filiformis

La souche de *Vitreoscilla filiformis* (ATCC 15551) est mise en culture selon le procédé décrit dans la demande de brevet WO-A-93-00741. Il s'agit d'un procédé de culture en continu. La culture s'effectue à 26°C durant au moins 48 heures jusqu'à l'obtention d'une concentration cellulaire convenable correspondant à une densité optique à 600 nm supérieure ou égale à 1,5. On repique la souche à 2 % V/V dans du milieu neuf durant 48 heures environ jusqu'à l'obtention d'une culture stable. Un Erlenmeyer de 1 litre contenant 200 ml de milieu neuf est alors ensemencé avec 4 ml de la culture précédente.
La culture en Erlenmeyer s'effectue à 26°C sur une table de culture agitée à 100 tours/minutes. Le pied de cuve ainsi obtenu sert d'inoculum à un fermenteur de 50 litres. La croissance s'effectue à 26°C, pH 7, 100 tours/minute et pO₂ ≥ 15 %.
Après 30 heures de croissance, la biomasse est transférée dans un fermenteur de 3000 litres utiles, pour être cultivée dans les mêmes conditions. Après 48 heures de croissance on récolte les cellules en continu. La biomasse est alors concentrée 50 fois environ par centrifugation. Les cellules obtenues sont alors congelées au fur et à mesure de la culture continue. Ces cellules peuvent être utilisées en l'état après décongélation (extrait cellulaire non stabilisé) ou bien être stabilisées par autoclavage à 121 °C durant 20 à 40 minutes (extrait cellulaire stabilisé). Les cellules ont alors éclaté durant la stérilisation en libérant le cytosol et en agglomérant les protéines ainsi que les parois. Le produit obtenu est alors biphasique.
La phase liquide surnageante peut être filtrée à 0,22 µm pour éliminer les particules ('surnageant').
L'extrait de bactérie, sous forme d'extrait cellulaire (stabilisé ou non) ou de surnageant, est utilisable en l'état (forme aqueuse) ou peut être lyophilisé suivant les techniques classiques (forme lyophilisée).

### Exemple 2 : Patch

On prépare un gel de composition suivante :

| | |
|---|---|
| - Méthyl sulfonyl méthane | 5 % |
| - Glycérine | 5 % |
| - Alginate de sodium | 10 % |
| - Alcool polyvinylique | 10 % |
| - Polyacrylate de sodium (Aronvis S de NIHON JUNYAKU) | 5 % |
| - Conservateurs | 1 % |
| - Eau | qsp 100% |

Après mélange des composants, le gel ainsi obtenu est enduit sur un support en non-tissé puis découpé pour former un patch.

### Exemple 3 : Patch

On prépare un gel de composition suivante :

| | |
|---|---|
| - Méthyl sulfonyl méthane | 5 % |
| - Glycérine | 5 % |
| - Alginate de sodium | 8,8 % |
| - Alcool polyvinylique | 11,3 % |
| - Copolymère acrylate | 7 % |
| - Carboxymethyl cellulose de sodium | 0,4 % |
| - Propylène glycol | 0,45 % |
| - Conservateurs | 0,15 % |
| - Eau | qsp 100% |

Après mélange des composants, le gel ainsi obtenu est enduit sur un support en non-tissé puis découpé pour former un patch.

Le patch de l'exemple 3 a été évalué sur un panel de 32 femmes ayant les ongles fragiles, abimés, cassants.
Ces femmes ont appliqué un pacth une fois par jour, au minimum pendant une demi heure, pendant 42 jours sur tous les ongles d'une seule main, les ongles de l'autre main n'étant pas traités (Témoin).
Au bout des 42 jours, les résultats ont été évalués sur les ongles traités et les ongles non traités, par mesure de la perte insensible en eau, échographie Haute Résolution et auto évaluation.

### 1/ Mesure de la perte insensible en eau (PIE)

Cette mesure s'effectue à l'aide d'un Tewamètre ® TM210 (Courage et Khazaka) basé sur l'évaluation du gradient de vapeur d'eau établi à la surface de l'ongle. Dépendante de l'humidité relative et de la température, la valeur de PIE est exprimée en masse d'eau évaporée par unité de surface et de temps.
Les mesures sont effectuées après une période d'acclimatation de 20 minutes, à une température maintenue entre 20 et 22°C et sous humidité relative entre 40 et 50%.
La sonde de l'appareil est appliquée perpendiculairement à l'ongle et la valeur moyenne est enregistrée lorsque les valeurs continues sont stables (après 30-45 secondes).
Les mesures sont réalisées sur l'ongle du pouce de chaque main, avant traitement, après 21 jours de traitement et après les 42 jours de traitement.

### Résultats :

| **Témoin** | | **Ongles traités** | |
|---|---|---|---|
| Après 21 jours de traitement | Après 42 jours de traitement | Après 21 jours de traitement | Après 42 jours de traitement |
| pas de variation significative de la perte insensible en eau | diminution significative de la PIE : -11.3 | diminution significative de la PIE : -15% | diminution significative de la PIE : -28.3% |

Une diminution significative de la PIE dès 21 jours et à 42 jours est observée pour les ongles traités.

### 2/ Evaluation de l'épaisseur de l'ongle par Echoqraphie Haute Résolution :

La mesure s'effectue à l'aide d'un Dermcup 2020 (2MT Medical-France) équipé d'une sonde opérant à une fréquence de 20MHz.
Les acquisitions échographiques Haute Résolution sont réalisées sur l'ongle du pouce de chaque main avant traitement (t0) puis après les 42 jours de traitement (t42).
Le champ de vision et le gain sont tenus constants pour un sujet donné à tous les temps de mesure. Trois images sont acquises successivement sur chaque site de mesures et à chaque temps de l'étude. La meilleure image enregistrée est ensuite analysée. Une série de 150 à 250 mesures est effectuée afin de définir une épaisseur moyenne de l'ongle pour les temps t0 et t42.

| **Témoin** | **Ongles traités (après42 jours)** |
|---|---|
| pas de variation significative de l'épaisseur de l'ongle | + 3 %. d'augmentation de l'épaisseur de l'ongle |

### On observe sur les ongles traités une augmentation significative de l'épaisseur des ongles.

### 3/ Auto évaluation :

59% des femmes ont constaté une augmentation de l'épaisseur des ongles de la main traitée avec le patch de l'exemple 3 à la fin de la période de traitement.
50% des femmes ont constaté une augmentation de la pousse des ongles traités.
69% des sujets ont jugé que le patch augmente la dureté des ongles.
69% des sujets ont jugé que le patch améliore l'état général des ongles.

### Exemple 4 : Patch

On prépare un patch de composition suivante :

| | |
|---|---|
| - Extrait de *Vitreoscilla filiformis* de l'exemple 1 5% | |
| - Méthyl sulfonyl méthane | 5 % |
| - Glycérine | 20 % |
| - Acide polyacrylique (Junlon PW 150 de NIHON JUNYAKU) | 4 % |
| - Tween 80 | 1 % |
| - Polyacrylate de sodium (Aronvis de NIHON JUNYAKU) | 5 % |
| - Acide tartarique | 1 % |
| - Conservateurs | 2 % |
| - Eau | qsp 100 % |

Après mélange des composants, le gel ainsi obtenu est enduit sur un support en non-tissé, puis découpé pour former un patch.

## Revendications

1. Dispositif transungéal comprenant au moins un actif choisi parmi un extrait de bactérie filamenteuse non photosynthétique non fructifiante, une dialkylsulfone et leur mélange.

2. Dispositif selon la revendication 1, **caractérisé en ce que** que ladite bactérie appartient au genre *Beggiatoa, Vitreoscilla, Flexithrix ou Leucothrix.*

3. Dispositif selon la revendication 1 ou 2, **caractérisé en ce que** ladite bactérie est une souche de *Vitreoscilla filiformis*, en particulier la souche ATCC15551.

4. Dispositif selon l'une quelconque des revendications 1 à 3, **caractérisée par le fait que** l'extrait de bactérie est un extrait cellulaire ou le surnageant dudit extrait, préférentiellement un extrait cellulaire ou une fraction active dudit extrait.

5. Dispositif l'une quelconque des revendications 1 à 4, **caractérisé en ce que** la dialkylsulfone présente la formule (I) suivante :
R₁-SO₂-R₂ (I)
dans laquelle R₁ et R₂ identiques ou différents représentent un groupe alkyle linéaire ou ramifié de 1 à 4 atomes de carbone, dans une composition cosmétique de soin des ongles, appliquée sur les ongles, pour favoriser la pousse des ongles.

6. Dispositif selon la revendication 5, dans laquelle le groupe alkyle de R₁ et R₂ est choisi indépendamment parmi les groupes méthyle, éthyle, isopropyle, n-propyle, n-butyle, isobutyle et tertiobutyle.

7. Dispositif selon la revendication 5 ou 6, dans laquelle R₁ et R₂ sont identiques.

8. Dispositif selon l'une des revendications 5 à 7, dans laquelle R₁ et R₂ représentent tous deux un groupe méthyle et le composé de formule (I) est la diméthylsulfone ou méthylsulfonylméthane (MSM).

9. Dispositif selon l'une quelconque des revendications 1 à 8, **caractérisé en ce que** le dispositif de délivrance transunguéal est sous forme de patch, de feuilles, de film composite ou de film solide, de dispositif augmentant l'absorption par un mécanisme actif.

10. Dispositif selon l'une quelconque des revendications 1 à 9 **caractérisé en ce qu'**il s'agit d'un patch choisi parmi les patchs à libération contrôlée, les patchs réservoirs, les patchs à effet de champ magnétique, les gels patchs à base forte teneur en eau (ou hydrogels), les gels patchs hydrophobes.

11. Dispositif selon l'une quelconque des revendications 1 à 10, **caractérisé en ce qu'**il se présente sous forme de patch comprenant un support souple et une matrice, de préférence adhésive, comprenant l'actif.

12. ispositif selon l'une quelconque des revendications 1 à 11, **caractérisé en ce que** la matrice comprend au moins un polymère.

13. Dispositif selon la revendication 12, **caractérisé en ce que** le polymère est choisi parmi les polymères naturels ou synthétiques et présentant un pouvoir adhésif.

14. Dispositif selon l'une des revendications précédentes, **caractérisée en ce qu'**il est un gel-patch hydrophile ou hydrogel dont la matrice comprend au moins un polymère hydrophile.

15. Dispositif selon la revendication 14, **caractérisé en ce que** le polymère hydrophile est choisi parmi les polymères et copolymères de vinyl pyrrolidone, l'acide acrylique, les dérivés, sels et co-polymères d'acide acrylique et leurs mélanges.

16. Dispositif selon l'une quelconque des revendications 13 à 15, **caractérisé en ce que** le polymère est présent en une proportion allant de 5 à 60% en poids par rapport au poids total de la matrice adhésive, de préférence de 10 à 40% en poids.

17. Dispositif selon l'une des revendications 13 à 16, **caractérisé en ce que** la matrice présente une teneur en eau allant de 30 à 90% en poids par rapport au poids total de la matrice.

18. Dispositif selon l'une quelconque des revendications 1 à 17, **caractérisée en ce que** l'actif est présent en une quantité allant de 0,1 % à 90% en poids par rapport au poids total de la matrice, préférentiellement allant de 0,5 à 50% en poids et encore plus préférentiellement de 1 à 30 % en poids.

19. Dispositif selon l'une des revendications 15 à 18, **caractérisé en ce que** la matrice comprend un solvant choisi parmi les alcools primaires, les polyols, les alcools polyvinyliques et leurs mélanges.

20. Dispositif selon la revendication 19, **caractérisé en ce que** la quantité de solvant va de 1 à 40 % en poids et de préférence de 5 à 30 % en poids par rapport au poids total de la matrice.

21. Dispositif selon l'une des revendications 14 à 19 **caractérisé en ce que** la matrice comprend un ou plusieurs hydrocolloïdes ou gélifiants hydrophiles.

22. Dispositif selon la revendication 21, **caractérisé en ce** les hydrocolloïdes ou gélifiants hydrophiles sont choisis dans le groupe formé par :
- la gomme de gellane ;
- la cellulose ou ses dérivés comme la carboxyméthylcellulose, l'hydroxypropyl-cellulose, la méthylcellulose, l'hydroxypropylméthylcellulose ou l'hydroxyéthyl-cellulose ainsi que les celluloses modifiées notamment par greffage de groupement alkyle ;
- les extraits d'algue tels que l'agar-agar, les carraghénanes, les alginates ;
- les extraits de graines tels que la gomme de caroube, la gomme de guar, les gommes de guar modifiées notamment par greffage de groupement alkyle ;
- les exsudats de plantes tels que la gomme arabique, la gomme karaya, la gomme adragante, la gomme de gatty ;
- les exsudats de micro-organismes tels que la gomme de xanthane ;
- les extraits de fruits tels que les pectines ;
- les dérivés du silicium tels que les hectorites synthétiques, les silicates d'aluminium et de magnésium
- et leurs mélanges.

23. Dispositif selon la revendication 21 ou 22, **caractérisé en ce que** l'hydrocolloïde est présent en une teneur allant de 5 à 40 % en poids et de préférence de 10 à 30 % en poids par rapport au poids total de la matrice.

24. Dispositif selon l'une quelconque des revendications 1 à 23, **caractérisé en ce que** le dispositif de délivrance transunguéal contient en outre au moins un composé accélérateur de pénétration.

25. Dispositif selon la revendication 24, **caractérisé en ce que** le composé accélérateur de pénétration est choisi dans le groupe comprenant les mono- et di-alcools non cycliques, l'acétate d'éthyle, l'acétate de butyle, le myristate d'isopropyle, les acides gras, les phospholipides, les terpènes, l'azone et dérivés, le propylène glycol et dérivés glycoliques, les cyclodextrines, l'octylsalicylate, le cyclopentadecanolide, les polysorbates, la polyvinylpyrrolidone et dérivés.

26. Dispositif selon la revendication 24 ou 25, **caractérisé en ce que** le composé accélérateur de pénétration est présent en une teneur allant de 0,01 % à 10 % en poids par rapport au poids de la matrice.

27. Dispositif selon l'une quelconque des revendications 1 à 26, **caractérisé en ce qu'**il contient en outre au moins un composé choisi parmi les gélifiants, les polymères adhésifs, les stabilisants, les conservateurs, les colorants, les pigments, les charges, les milieux solvant aqueux ou organique, les parfums, les ions , les initiateurs de polymérisation et leurs mélanges.

28. Procédé de traitement cosmétique consistant à appliquer sur l'ongle un dispositif transunguéal selon l'une quelconque des revendications 1 à 27.

29. Utilisation d'un dispositif transungéal comprenant au moins un actif choisi parmi un extrait de bactérie filamenteuse non photosynthétique non fructifiante, une dialkylsulfone et leur mélange, pour renforcer les ongles et en particulier pour durcir les ongles et/ou augmenter leur résistance à la cassure et/ou au dédoublement et/ou augmenter l'épaisseur des ongles, et/ou stimuler la pousse et/ou la repousse des ongles.

30. Utilisation d'un dispositif transungéal comprenant au moins un actif choisi parmi un extrait de bactérie filamenteuse non photosynthétique non fructifiante, une dialkylsulfone et leur mélange, pour limiter la perte insensible en eau des ongles, favoriser l'hydratation des ongles et/ou favoriser la pénétration de l'actif dans l'ongle.
